(19) Europäisches Patentamt
European Patent Office
Office européen des brevets

(11) Veröffentlichungsnummer: **0 518 270 A1**

(12) # EUROPÄISCHE PATENTANMELDUNG

(21) Anmeldenummer: **92109711.9**

(51) Int. Cl.[5]: **C07H 3/06**, C07H 3/04

(22) Anmeldetag: **10.06.92**

(30) Priorität: **13.06.91 DE 4119472**

(43) Veröffentlichungstag der Anmeldung:
**16.12.92 Patentblatt 92/51**

(84) Benannte Vertragsstaaten:
**AT BE CH DE DK ES FR GB GR IT LI LU NL PT SE**

(71) Anmelder: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Kinzy, Willy, Dr.**
**Schlossstrasse 19b**
**W-7854 Inzlingen(DE)**

(54) **Verfahren zur Herstellung von Zuckerepitopen.**

(57) Die Erfindung betrifft ein neues Verfahren zur stereoselektiven Herstellung von Gal-α(1-3)/Gal-β(1-4)/GlcNac-Trisacchariden der Formel

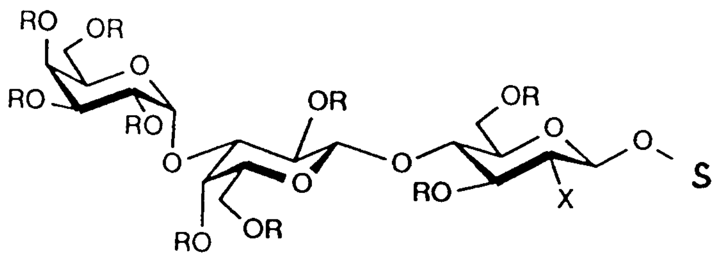

und von β(1-3)-verknüpften Oligolactosaminen der Formel

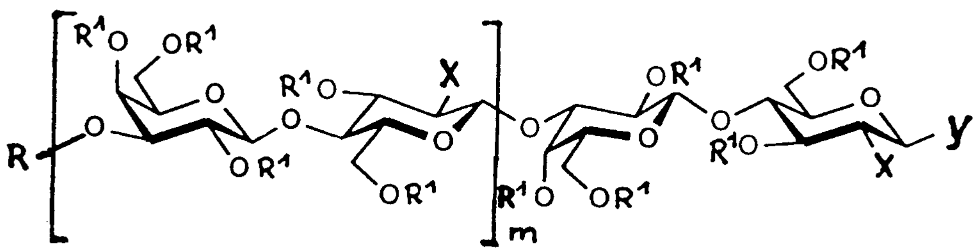

über partiell alkylierte Glykale der Formel

EP 0 518 270 A1

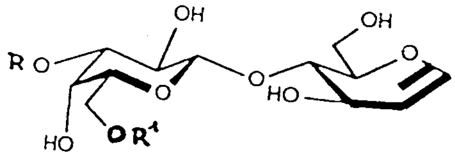

und andere neue Zwischenprodukte, worin je nach Formel

R H, $R'CH_2$-, Allyl,
$R^1$ H, $R'CH_2$-, Allyl oder R'CO-,
R' H, $C_1$ bis $C_3$ -Alkyl oder (un)substituiertes Phenyl,
S einen Spacer, vorzugsweise der Formel $-(CH_2)_n-COOR''$,
R'' Alkyl mit 1 bis 4 C-Atomen,
X $N_3$ oder $-NHCOCH_3$,
Y $\beta$-$OSiR_2{}^3$ oder $\alpha$-$OC(=NH)CCl_3$,
$R^2$ $C_1$ bis $C_4$-Alkyl oder Phenyl

und

m, n eine ganze Zahl von 1 bis 4 bzw. 4 bis 12

bedeuten.

Die Erfindung betrifft ein neues Verfahren zur stereoselektiven Herstellung von Gal-α(1-3)/Gal-β(1-4)-/GlcNac-Trisacchariden und von β(1-3)-verknüpften Oligolactosaminen über partiell alkylierte Glykale.

Die Erfindung betrifft im speziellen ein Verfahren zur stereoselektiven Herstellung von α(1-3), β(1-4) konfigurierten Trisacchariden der Formel I,

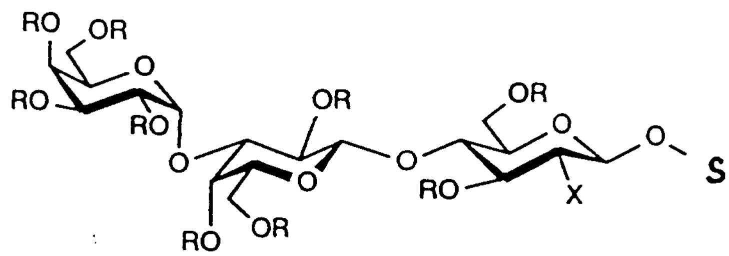

I

worin

- R H, R'$CH_2$-,
- R' H oder Alkyl mit 1 bis 3 C-Atomen oder unsubstituiertes oder mit Halogen, OH, Alkyl oder O-Alkyl substituiertes Phenyl,
- S einen Spacer und
- X $N_3$ oder -$NHCOCH_3$

bedeuten,

aus Lactose, dadurch gekennzeichnet, daß man

i. Lactose zu D-Lactal umsetzt;

ii. D-Lactal zu den neuen Lactalderivaten der Formel II,

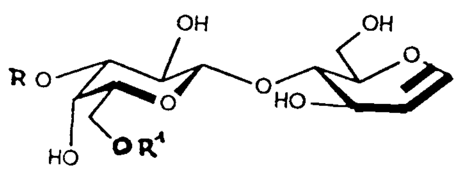

II

worin

- R R'$CH_2$- oder -$CH_2$-$CH=CH_2$,
- $R^1$ H, R'$CH_2$-, $CH_2$-$CH=CH_2$ oder R'-C(O)- und
- R' H, Alkyl mit 1 bis 3 C-Atomen, oder

unsubstituiertes oder mit Halogen, OH, Alkyl oder O-Alkyl substituiertes Phenyl

bedeuten,

partiell alkyliert;

iii. die Verbindung der Formel II, worin R -$CH_2$-$CH=CH_2$ und $R^1$ H bedeuten, zu mit R'$CH_2$- vollständig geschützten Mono-Allyl-Verbindungen umsetzt;

iv. diese stereoselektiv durch Azidonitratisierung zu den Azido-Lactosederivaten der Formel III,

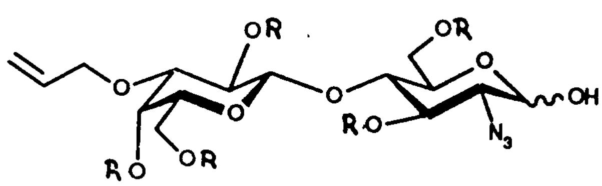

III

worin R R'$CH_2$- bedeutet und R' die angegebene Bedeutung hat, umsetzt;

v. das Gluco-Isomer der Verbindungen der Formel III entweder zu den Azido-Lactosederivaten der Formel IV,

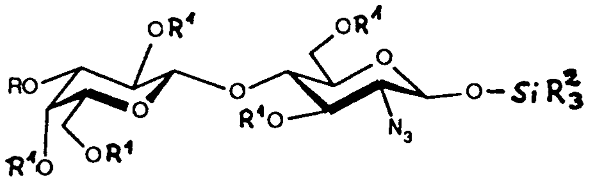

IV

worin die zunächst entstehende Allylverbindung durch Abspaltung der Allylgruppe zur OH-freien Verbindung umgesetzt wird und der Silylrest β-konfiguriert ist,
oder zu aktivierten Azido-Lactosederivaten der Formel V,

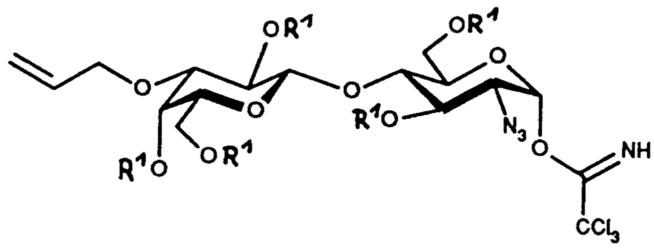

V

worin der Trichloracetimidat-Rest (O-C(=NH)$CCl_3$) vorzugsweise α-konfiguriert ist, stereoselektiv umsetzt, wobei in den Formeln IV und V $R^1$ R'$CH_2$-, R Allyl oder H und $R^2$ $C_1$ bis $C_4$-Alkyl oder Phenyl bedeuten und R' die angegebenen Bedeutung hat;
vi. die Disaccharide der Formel IV oder V unter Einführung eines Galactopyranosyl-Restes und Substitution des glykosidischen Restes des Glucopyranosylringes durch einen Spacerrest -O-S zu den Verbindungen der Formel I,
worin
R R'$CH_2$ ,X $N_3$ und S einen Spacer bedeuten, R' die angegebene Bedeutung hat und der Spacerrest β-konfiguriert ist, umsetzt, und
vii. gegebenenfalls die Azido-Gruppe zur -$NHCOCH_3$ und die OR-Reste zu OH-Gruppen reduziert.

Die Erfindung betrifft ferner die neuen Lactalderivate der Formel II.

Die Erfindung betrifft weiter die neuen α(1-3), β(1-4) konfigurierten Trisaccharide der Formel VI,

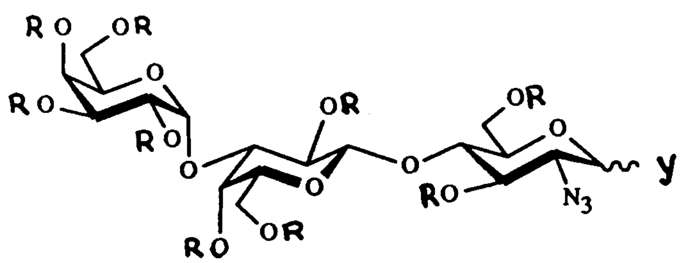

VI

worin R R'$CH_2$-, Y β-$OSiR^2_3$ oder α-OC(NH)$CCL_3$ und R' und $R^2$ die angegebenen Bedeutungen haben.

Die Erfindung betrifft außerdem die neuen β(1-4) konfigurierten Disaccharide der Foreml VII,

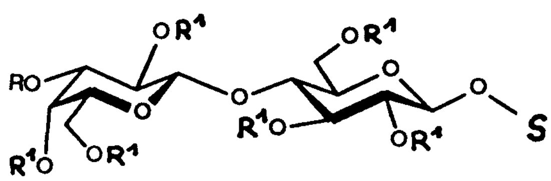

VII

worin S einen Spacer der Formel -$(CH_2)_n$-COOR'', R'' $C_1$ bis $C_4$-Alkyl, n eine ganze Zahl von 4 bis 12, R Allyl oder H, $R^1$ R'$CH_2$- und X $N_3$ oder -$NHCOCH_3$ bedeuten und R' die angegebene Bedeutung hat.

Letztlich betrifft die Erfindung die neuen $\beta$-verknüpften Oligolactosamine der Formel VIII,

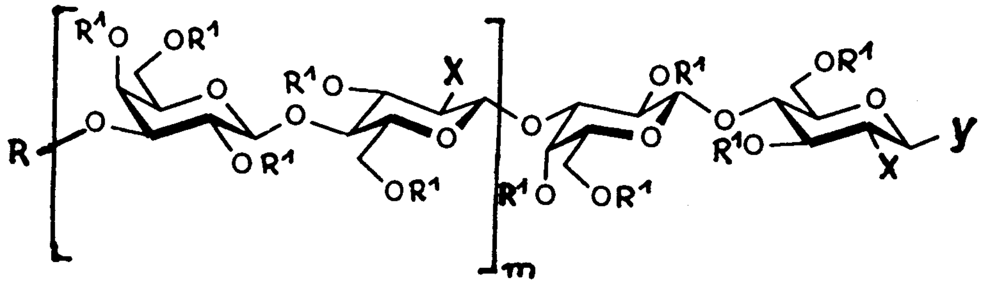

VIII

worin

| | |
|---|---|
| R | H, Allyl, |
| $R^1$ | H, $R'CH_2$-, |
| X | $N_3$, $-NHCOCH_3$, |
| Y | $\beta$-$OSiR^2_3$, $\alpha$-O-C(=NH)$CCl_3$ |
| $R^2$ | $C_1$ bis $C_4$-Alkyl oder Phenyl und |
| m | eine ganze Zahl von 1 bis 4 |

bedeuten und und R' die angegebene Bedeutung hat.

Verbindungen der Formel I sind an sich bekannt (Garegg et al. (1985), Carbohydrate Res. 136, 207-213). Die Trisaccharidkomponenten dieser Verbindungen stellen Epitope dar, die auf humanen Tumorzellen exprimiert werden, während normale Zellen diese Epitope nicht oder nur in sehr geringen Mengen produzieren (z.B. Galili (1983), Lancet 2, 358-360). Entsprechend können solche Epitope als Hapten fungieren und Antikörper initiieren, die als potentielle Kandidaten für die therapeutische Behandlung von Tumoren bzw. Tumormetastasen oder zur Stimulation der körpereigenen Abwehr gegen Tumorzellen eingesetzt werden können (z.B. Castronovo et al. (1989), J. Natl. Cancer Inst. 81 (3), 212-216).

Die Bereitstellung der Verbindungen der Formel I, in denen ein Spacer die Ankopplung an eine Proteinmatrix ermöglicht, stellt also angesichts ihrer Anwendung auf dem medizinischen Sektor ein lohnendes Ziel dar. Bislang konnte eine spezielle Verbindung der Formel I hergestellt werden (Garegg et al., l.c.). Die bekannte Synthese verläuft aber über sehr viele Zwischenstufen, wodurch sie für Einsätze im technischen Mabstab nur begrenzt eingesetzt werden kann. Durch die vielen Zwischenstufen ist naturgemäß die Gesamtausbeute nicht sehr hoch. Auf der anderen Seite stellen derartige Synthesen hohe Anforderungen an Stereoselektivität bzw. Stereospezifität.

Es wurde nun gefunden, daß die Verbindungen der Formel I in einer effizienten, stereoselektiven und relativ kurzen Synthese leicht aus kommerziell erhältlicher Lactose hergestellt werden können, wenn man die Synthese über die bisher nicht verfügbaren partiell alkylierten Glykale der Formel II führt, die Doppelbindung der geschützten Glykale azidonitratisiert und durch Einführung der leicht wieder abspaltbaren Trichloracetimidat- bzw. tert.-Butyldimethylsilyl-Gruppe ein entsprechendes Donor- bzw. Akzeptormolekül herstellt, das mit einem entsprechenden Monosaccharid zu den gewünschten Verbindungen der Formel I oder mit dem korrespondierenden Donor-/Akzeptor-Disaccharid unter Einwirkung von vorzugsweise Trimethylsilyltrifluormethansulfonat zu neuen $\beta$(1-3)-verknüpften Tetra- bzw. Oligolactosaminen der Formel VIII reagiert. Letztere stellen wichtige Determinanten dar, die ebenfalls als Tumorantigene bekannt sind und somit in analoger Weise eingesetzt werden können, wie die Verbindungen der Formel I.

Es wurde insbesondere gefunden, daß die erfindungsgemäßen Verbindungen der Formel II in besonders guten Ausbeuten unter Einwirkung von Dibutyl-zinnoxid aus D-Lactal hergestellt werden können. Es wurde ferner gefunden, daß neben den Verbindungen der Formel II auch die Verbindungen der Formeln VI und VII wertvolle neue Zwischenprodukte bei der Synthese der Verbindungen der Formel I darstellen.

Das erfindungsgemäße Verfahren weist neben der ausgezeichneten Stereoselektivität, insbesondere einiger Schritte, lediglich 15 Syntheseschritte ausgehend von Lactose auf im Vergleich zu 23 Syntheseschritten bei der Anwendung des Verfahrens von Garegg et al. (l.c.).

Vor- und nachstehend haben die Reste R, R',$R^1$, R'', X, Y und S die angegeben Bedeutungen, sofern nicht ausdrücklich etwas anderes angegeben ist.

R und $R^1$ bedeuten je nach Formeln I bis VIII H, $R'CH_2$-, R'C(O)- oder $-CH_2$-CH=$CH_2$; R' bedeutet H oder Alkyl mit 1 bis 3 C-Atomen oder unsubstituiertes oder mit Halogen, OH, Alkyl oder O-Alkyl substituiertes Phenyl. Bedeutet R' Alkyl so sind dies im einzelnen Methyl, Ethyl, n-Propyl oder Isopropyl.

Im Falle von $R'CH_2$- bedeutet R' vorzugsweise unsubstituiertes oder substituiertes, vorzugsweise jedoch

unsubstituiertes Phenyl. Bedeutet R' aber substituiertes Phenyl, so kann Phenyl einfach oder mehrfach substituiert sein. Mit Ausnahme von Mesityl ist es aber vorzugsweise einfach substituiert. Als Substituenten eignen sich F, Cl, Br, OH, Alkyl mit 1 bis 3 C-Atomen oder O-Alkyl mit 1 bis 3 C-Atomen, vorzugsweise aber F, OH oder Methyl.

Im Falle von R'C(O)- bedeutet R' vorzugsweise Methyl oder Phenyl.

X bedeutet $N_3$ oder $-NHCOCH_3$, vorzugsweise aber $-NHCOCH_3$;

Y bedeutet $\beta$-$OSiR^2_3$ im Falle eines (Glycosyl-)Akzeptormoleküls oder $\alpha$-O-C(=NH)$CCl_3$ im Falle eines (Glycosyl-) Donormoleküls.

$R^2$ bedeutet C1 bis C4-Alkyl oder Phenyl, wobei die Reste R2 gleich oder verschieden sein können. Vorzugsweise bedeutet $-SiR^2_3$ aber tert-Butyldimethylsilyl (TBDMS) oder Texyldimethylsilyl (TDMS).

S bedeutet einen Spacerrest $-(CH_2)_n$-COOR", worin R" $C_1$ bis $C_4$-Alkyl und n eine ganze Zahl von 4 bis 12 bedeuten. Alkyl kann geradkettig oder verzweigt sein und bedeutet im einzelnen Methyl, Ethyl, n-Propyl, Isopropyl, n-Butyl, Isobutyl, sec. und tert. Butyl. Vorzugsweise bedeutet R" Methyl oder Ethyl. n bedeutet vorzugsweise 6 bis 10, insbesondere aber 8. Der besonders bevorzugte Spacerrest ist $-(CH_2)_8$-COOEt. Der Spacerrest ist glycosidisch an das Di- oder Trisaccharid gebunden. Vorzugsweise wird er als S-OH in den jeweiligen Zuckerrest eingeführt. Über die COO-Gruppe des als Abstandhalter fungierenden Spacers kann die Bindung an ein bestimmtes Träger-Proteinmolekül erfolgen. Hierdurch kann das Hapten (Zuckerepitop) seine antigene bzw. immunogene Wirkung entfalten.

Im folgenden wird das erfindungsgemäße Verfahren in allgemeiner Form beschrieben. Verfahrensschritte, die Standardmethoden der Kohlenhydratchemie entsprechen, wie zum Beispiel die Einführung und Abspaltung geeigneter Schutzgruppen, sind nicht näher erläutert. Diesbezüglich wird auf die Standardliteratur verwiesen (z.B. T.W. Greene, Protective Groups in Organic Synthesis, 1981, John Wiley & Sons; Methods in Carbohydrate Chemistry, Vol. I-VIII, Academic Press).

Zunächst wird Lactose in bekannter Weise in D-Lactal überführt (Kent et al. (1977), J. Fluorine Chem. 10, 455-478). D-Lactal entspricht einer Verbindung der Formel II, bei der alle Reste R, $R^1$ und R' H bedeuten. Die bei dem erfindungsgemäßen Verfahren bevorzugte Anwendung der Dibutyl-zinn-Methode (Wagner et al. (1974), J. Org Chem. 39, 24; Auge et al. (1976), J. Chem. Soc. Chem. Commun., 375-376, Nashed (1977), Carbohydr. Res. 56, 325-336) führt zu Verbindungen der Formel II, bei denen lediglich der Rest R alkyliert ist, während die Reste $OR^1$ als freie OH-Gruppen vorliegen. Bevorzugt werden Allylsubstrate eingesetzt, so daß die monoallylierte Verbindung der Formel II (R = $-CH_2-CH=CH_2$, $R^1$=H) gebildet wird. Bei dieser Reaktion entstehen als Nebenprodukte in geringen Ausbeuten die di-O-alkylierten Derivate der Verbindungen der Formel II. Um die Regiochemie der partiellen Alkylierung zu bestimmen, werden die Reaktionsprodukte in an sich bekannter Weise peracetyliert bzw. mit Trichloracetylisocyanat behandelt und mittels NMR-Spektroskopie untersucht. Man erhält z.B. die bevorzugte Verbindung der Formel II (R = $-CH_2-CH=CH_2$,$R^1$=H) nach chromatographischer Aufreinigung als hochkristallinen Feststoff in einer Ausbeute von 58 bis 65 %. Das verwendete Lösungsmittel kann dabei den Anteil des Nebenproduktes beeinflussen. So erhält man höhere Ausbeuten an der di-O-allylierten Verbindung der Formel II, wenn man Toluol statt Benzol einsetzt.

Die Bildung der mono- bzw. dialkylierten Lactale der Formel II mit Hilfe der Stannylierungs-Methode steht überraschenderweise im deutlichen Gegensatz zu den Ergebnissen der Alkylierung von anderen Glykalen mit Hilfe der gleichen Methode (Mereyala et al. (1989), Carbohydr. Res. 187, 154-158).

Die freien OH-Gruppen der Verbindungen der Formel II werden anschließend alkyliert vorzugsweise benzyliert. Dies geschieht nach Standardmethoden beispielsweise mit Alkyl(Benzyl)-Bromid in Tetrahydrofuran. Man erhält in nahezu quantitativer Ausbeute die vollständig geschützten Lactale der Formel II. Vorzugsweise wird das mono-allylierte Lactal der Formel II eingesetzt.

Es folgt nun die Azidonitratisierung der vollständig geschützten Verbindungen in an sich bekannter Weise (Lemieux et al. (1979), Can. J. Chem. 57, 1244-1251). Nach Spaltung der intermediär gebildeten Nitratester erhält man die 1-OH-freien Azido-lactosederivate der Formel III, vorzugsweise die entsprechenden monoallylierten Verbindungen.

Zur Herstellung des Akzeptorbausteins IV werden die vorzugsweise monoallylierten Verbindungen der Formel III nach Standard-methoden beispielsweise mit tert.-Butyldimethylsilylchlorid (TBDMSCl) an der anomeren OH-Gruppe silyliert.

Die Reaktion führt stereoselektiv zu den $\beta$-silylierten Verbindungen, die in einem Epimerengemisch (gluco / manno : 1.5 bis 2.5 : 1) vorliegen. Das Epimerengemisch kann mittels Chromatographie, beispielsweise mit Mitteldruck-flüssigkeitschromatographie (MPLC) nach Standardmethoden (z.B. Jung et al. (1989), Liebigs Ann. Chem., 1099-1106) getrennt und aufgereinigt werden. Für die weiteren Syntheseschritt

eignen sich bevorzugt die gluco-konfigurieten Epimere.

Die Allyl-Gruppe der β-konfigurierten Silylderivate der Formel IV wird selektiv in eine freie OH-Gruppe beispielsweise unter Verwendung des Wilkinson Katalysators (Maranduba et al. (1985), Carbohydr. Res. 135, 330) und anschließender Behandlung mit Quecksilber-II-Oxid / Quecksilber-II-Chlorid zur Spaltung des intermediär gebildeten Propenylderivats umgewandelt. Man erhält so bevorzugt das Lactosaminderivat der Formel IV, worin R H bedeutet, in ausgezeichneter Ausbeute.

Die wie beschrieben erhältlichen Akzeptorbausteine der Formel IV werden nun mit aus der Literatur zugänglichem per-O-benzylierten oder anderweitig geschütztem α-Galactopyranosyl-trichloracetimidat (Wegmann et al. (1987), J. Carbohydr. Chem. 6 (3), 357-375) vorzugsweise in Gegenwart von Trimethylsilyl-trifluormethansulfonat stereoselektiv zu den α(1-3), β(1-4) konfigurierten, neuen Trisacchariden der Formel VI, worin Y $-OSiR^2_3$, vorzugsweise aber -OTBDMS bedeutet und β-konfiguriert ist, in guten Ausbeuten zwischen 75 und 80 % umgesetzt. Auch andere gebräuchliche Lewis-Säuren wie z.B. Bortrifluorid-Diethylether, Zinn(II)-chlorid, Zinkchlorid, Zinkchlorid-Etherat, Titantetrachlorid oder p-Toluolsulfonsäure sind erfindungsgemäß in katalytischen Mengen geeignet. Der stereoselektive Austausch der glycosidischen β-O-$SiR^2_3$- bzw. β-O-TBDMS-Gruppe durch die α-konfigurierte Trichloracetimidat-Gruppe erfolgt durch Abspaltung der Akzeptorgruppe und Bildung der freien 1-OH-Gruppe durch Behandlung mit vorzugsweise Tetrabutylammonium-fluorid oder anderen bekannten für diese Zwecke geeigneten sauer wirkenden Reagenzien, gefolgt von der Umsetzung mit Trichloracetonitril unter basischen Bedingungen, z.B. mit Hilfe von Natriumhydrid oder auch DBU, $K_2CO_3$ oder $Cs_2CO_3$. Dabei entstehen ausschlieblich die neuen, α-konfigurierten Trichloracetimidate der Verbindungen der Formel VI in Ausbeuten zwischen 70 und 80 %.

Die Trichloracetimidate der Verbindungen der Formel VI werden nun mit dem Spacer-Reagenz S-OH stereospezifisch zu den β-glycosidisch verknüpften Azido-Verbindungen der Formel I in Gegenwart eines unpolaren Lösungsmittels, vorzugsweise eines Dichlormethan/n-Hexan-Gemisches, und eines Katalysators, vorzugsweise Bortrifluorid-Diethylether, in guten Ausbeuten (70 bis 80 %) umgesetzt.

Die Azido-Gruppe kann nun in an sich bekannter Weise beispielsweise mit Natriumborhydrid reduziert und anschließend mit z.B. Essigsäureanhydrid acetyliert werden (X = $-NHCOCH_3$). Die hydrogenolytische Dealkylierung, insbesondere Debenzylierung, mit beispielsweise Palladium / Kohlenstoff in vorzugsweise Etylacetat / Etanol / Wasser / Essigsäure führt schließlich zu der Verbindung der Formel I, worin R H und X $-NHCOCH_3$ bedeuten, und die im besonderen Maße als Hapten eingesetzt werden kann.

In einer alternativen Ausführungsform des erfindungsgemäßen Verfahrens kann die Synthese auch mit Hilfe der Verbindungen der Formel V weitergeführt werden. Die Verbindungen können aus den Verbindungen der Formel III durch Umsetzung mit Trichloracetonitril und Natriumhydrid in an sich bekannter Weise erhalten werden. Dabei werden vorwiegend die α-Trichloracetimidate der entsprechenden Disaccharide gebildet.

Das entstandene Epimerengemisch von Gluco/manno-Derivaten kann wie oben geschildert, leicht aufgetrennt werden. Die Gluco-isomere werden dem weiteren Reaktionverlauf zugrundegelegt.

Bei dieser Verfahrensvariante wird anschließend bereits auf der Stufe der Disaccharide der Spacerrest S nach der oben beschriebenen Vorgehensweise eingeführt. Man erhält die neuen Derivate der Formel VII, worin der Spacerrest wieder β-konfiguriert ist.

Die Allyl-Gruppe der geschützten Disaccharid-Spacer-Derivate der Formel VII wird dann, wie oben geschildert, zur freien OH-Gruppe reduziert. Die so gewonnen Verbindungen der Formel VII werden mit per-O-benzyliertem oder anderweitig geschütztem α-Galactopyranosyl-trichloracetimidat, wie oben beschrieben, zu den gegebenenfalls dealkylierten, $-NHCOCH_3$-substituierten (an der ursprünglichen Azido-Position) Trisacchariden der Formel I umgesetzt. Neben den Verbindungen der Formel I, die α(1-3), β(1-4)-konfiguriert sind und in einer Ausbeute von 60-70 % erhalten werden können, entstehen bei dieser Verfahrensvariante zu 16-20 % auch die entsprechenden β(1-3), β(1-4)-konfigurierten Trisaccharide.

Das Verfahren über die Verbindungen der Formel IV ist jedoch bevorzugt, da einerseits keine β(1-3), β-(1-4)-konfigurierten Trisaccharide sondern ausschließlich die gewünschten α(1-3), β(1-4)-konfigurierten Trisaccharide entstehen, andererseits die Ausbeuten des Reaktionsschrittes, der die Ankopplung des Spacerrestes betrifft, größer sind.

Die aktivierten Azido-Lactosederivate der Formel V und die Lactosamin-Akzeptorderivate der Formel IV stellen wichtige Zwischenprodukte bei der stereoselektiven Synthese von neuen β(1-3)-verknüpften Oligolactosaminen der Formel VIII dar. Derartige Lactosamine können als mögliche Determinanten auf Tumorantigenen eine wichtige Rolle spielen.

Erfindungsgemäß können die Verbindungen der Formel VIII hergestellt werden durch direkte Umsetzung der Verbindungen der Formel IV, worin R H bedeutet, mit denen der Formel V in Gegenwart von vorzugsweise Trimethylsilyl-trifluormethansulfonat z. B. in einer Mischung von Dichlormethan und n-Hexan. Neben Trimethylsilyl-trifluormethansulfat sind auch andere, oben genannte, gebräuchliche Lewis-Säuren in

katalytischen Mengen geeignet. Zunächst entstehen ausschließlich die β(1-3)-verknüpften Tetrasaccharide der Formel VIII, worin Y β-O-TBDMS, R Allyl und m = 1 bedeuten, in einer Ausbeute zwischen 70 und 80 %. Durch eine erneute, oben beschriebene Deallylierung und Umsetzung mit einem weiteren Molekül der Formel V erhält man das entsprechende Hexasaccharid (m = 2). In analoger Weise kann man so das entsprechende Octa- (m = 3) und Decasaccharid (m = 4) herstellen.

Das erfindungsgemäße Verfahren zur Herstellung der wertvollen Verbindungen der Formel I zeichnet sich zusammengefaßt durch folgende Besonderheiten aus:

- der Einsatz der Stannylierungsmethode gewährleistet im Gegensatz zu den bislang bekannten analogen Synthesen die Bereitstellung von partiell alkylierten, geschützten neuen D-Lactalen, die wichtige Zwischenprodukte darstellen;
- die Anwendung der Trichloracetimidat-Methode ermöglicht eine extrem hohe Stereoselektivität einzelner Reaktionsschritte;
- der Spacer wird in der bevorzugten Variante des erfindungsgemäßen Verfahrens erst in einer späten Phase in das Molekül eingeführt (im Gegensatz zum unmittelbaren Stand der Technik nach Garegg et al., l.c.); dadurch können Ausbeuten und Stereoselektivität verbessert werden.
- das Verfahren ermöglicht überdies den leichten Zugang zu für die Medizin wertvollen, neuen β(1-3)-verknüpften Oligolactosaminen.

Im folgenden wird das erfindungsgemäße Verfahren anhand konkreter Beispiele erläutert.

Verwendetes chromatographisches Material:

1. Dünnschichtchromatographie: Kieselgel 60 F-254 (E. Merck, Darmstadt, Germany), Detektion mit 15%iger Schwefelsäure.
2. Säulenchromatographie: Kieselgel 60, 0.063-0.200 nm (E. Merck, Darmstadt, Germany).
3. Mitteldruckchromatographie (MPLC): Kieselgel LiChroPrep® Si 60, 15-25 µm.
4. HPLC: Kieselgel LC-8 (Shimadzu, Japan)

## Beispiel 1:

Eine Mischung aus D-Lactal (600 mg, 1.94 mmol) und Dibutylzinnoxid (1.0 g, 4 mmol) in trockenem Benzol wird etwa 20 Stunden lang bei Rückflußtemperatur erhitzt. Nach Einengung der Lösung auf etwa 20 ml werden Tetrabutylammoniumjodid (700 mg, 2.0 mmol) und Benzylbromid (1 ml, 8.0 mmol) hinzugefügt und die Lösung wird für etwa weitere 3 Stunden unter Rückfluß gekocht. Das Lösungsmittel wird bei $10^{-2}$ Torr abgezogen und der gelbe Rückstand wird über eine Kieselgelsäule gereinigt (Eluent: Ethylacetat / Methanol 9 : 1). Die Fraktion mit einem $R_F$-Wert von 0.39 wird zur Trockene eingeengt und ergibt 400 mg (52 %) eines farblosen Sirups, der aus Ethylacetat auskristallisiert.

Man erhält

**4-O(3-O-Benzyl-β-D-galactopyranosyl)-D-arabino-hex-1-enitol,**

eine Verbindung der Formel II.

$R_F$-Wert (TLC): 0.39 (Ethylacetat/Methanol 9:1)

Mp.: 175 °C; $[\alpha]_D$ = +42.9 (c = 1, Chloroform).

## Beispiel 2:

Eine Mischung von D-Lactal (308 mg, 1.0 mmol) und Dibutylzinnoxid (500 mg, 2.0 mmol) in trockenem Toluol wird etwa 17 Stunden lang bei Rückflußtemperatur erhitzt. Nach Einengung der Lösung auf etwa 20 ml werden Tetrabutylammoniumjodid (369 mg, 1.0 mmol) und Benzylbromid (0.5 ml, 4.0 mmol) hinzugefügt und die Lösung wird für etwa weitere 4 Stunden unter Rückfluß gekocht. Das Lösungsmittel wird bei $10^{-2}$ Torr abgezogen und der gelbe Rückstand wird über eine Kieselgelsäule gereinigt (Gradienten Eluent: Ethylacetat/Methanol 1 : 0 → 9 : 1). Die Hauptfraktion ($R_F$-Wert: 0.70, Ethylacetat/Methanol 9 : 1) enthält 180 mg (33 %)

**4-O(3,6-Di-O-benzyl-β-D-galactopyranosyl)-D-arabino-hex-1-enitol,**

eine Verbindung der Formel II. Die folgende kleinere Fraktion enthält 90 mg (23 %) der Verbindung, die gemäß Beispiel 1 hergestellt wurde.

## Beispiel 3:

Eine Mischung von D-Lactal (40 g, 0.130 mol) und Dibutylzinnoxid (64.6 g, 0.259 mol) in trockenem Benzol (1100 ml) wird etwa 17 Stunden lang bei Rückflußtemperatur erhitzt. Zur vollständigen Reaktion wird ein Molekularsieb (4 A°) hinzugefügt und die Mischung wird für weitere 2 Stunden unter Rückfluß gekocht.

Nachdem die Lösung auf etwa 1/3 des Volumens eingengt worden ist, werden Tetrabutylammoniumjodid (47.8 g, 0.130 mol) und Allylbromid (31.4 g, 0.259 mol) dem Ansatz hinzugefügt. Nach 4 Stunden unter Rückfluß erfolgt keine weitere Reaktion (TLC: Ethylacetat/Methanol 9 : 1). Das Lösungsmittel wird abgezogen und der Rückstand in Dichlormethan aufgenommen. Die organische Phase wird mehrmals mit Wasser extrahiert und die wäßrige Phase eingeengt. Man erhält einen gelben Rückstand (69 g), der mittels Kieselgel-Säulenchromatographie (Ethylacetat/Methanol 9:1) aufgereinigt wird. Die als erste eluierte Fraktion ($R_F$ = 0.67) enthält die Di-allylverbindung der Formel II und zwar

**4-O(3,6-Di-O-allyl-β-D-galactopyranosyl)-D-arabino-hex-1-enitol**

(7 g, 12 %). $[\alpha]_D$ = +40.9 (c = 1, Chloroform).

Die Hauptfraktion ($R_F$ = 0.31, (28 g, 60 %) enthält

**4-O(3-O-Allyl-β-D-galactopyranosyl)-D-arabino-hex-1-enitol,**

eine Verbindung der Formel II, die sich aus Ethylacetat umkristallisieren läßt.

$R_F$-Wert (TLC): 0.45 (Ethylacetat/Methanol 85:15)

Mp.: 148 - 149 °C; $[\alpha]_D$ = +41.3 (c = 1, Chloroform).

**Beispiel 4:**

Zu einer Suspension des nach Beispiel 3 hergestellten Monoallylderivates (12.0 g, 34.4 mmol) in trockenem Tetrahydrofuran (750 ml) wird unter einer Argonschutzgas-Atmosphere portionsweise Natriumhydrid (6 x 1.65 g, 69 mmol) und Benzylbromid (6 x 10.2 ml, 86 mmol) unter kräftigem Rühren hinzugefügt. Die Mischung wird auf Rückflußtemperatur erhitzt und es werden katalytische Mengen Tetrabutylammoniumjodid und 18-Kroneether-6 hinzugegeben. Nach ca. 19 Stunden bei 60 °C unter kontrollierter Wasserstoffreisetzung kommt die Reaktion zum Stillstand (Überprüfung der Reaktion mit TLC). Die Mischung wird auf Raumtemperatur abgekühlt und gefiltert. Zu dem Filtrat wird gestobenes Eis hinzugegeben und die Lösung wird mehrmals mit Diethylether extrahiert. Die organische Phase wird neutralisiert, mehrmals mit Kochsalzlösung gewaschen, über Magnesiumsulfat getrocknet und eingedampft. Der Rückstand wird über Kieselgel aufgereinigt (Eluent: Petrolether dann Ethylacetat), um flüssige Nebenprodukte zu entfernen, und schließlich mittels Flashchromatographie (Petrolether / Ethylacetat 85 : 15) weiter aufgereinigt. Man erhält als gelben Sirup 26 g (95 %)

**3,6-Di-O-benzyl-4-O(2,4,6-tri-O-benzyl-3-O-allyl-β-D-galactopyranosyl)-D-arabino-hex-1-enitol,**

eine Verbindung der Formel II.

$R_F$-Wert (TLC): 0.40 (Petrolether/Ethylacetat 8 : 2);

$[\alpha]_D$ = -13.8 (c = 1, Chloroform).

**Beispiel 5:**

Eine Lösung von der in Beispiel 4 hergestellten Verbindung (4.0 g, 5 mmol) in trockenem Acetonitril (60 ml) wird unter Argongas-Atmosphere auf -30 °C abgekühlt. Cerammoniumnitrat (6.85 g, 12.5 mmol) und Natriumazid (0.41 g, 6.26 mmol) werden unter kräftigem Rühren hinzugefügt. Nach etwa 17 Stunden bei -30 °C und weiteren 3 Stunden bei -20 °C wird die Suspension gefiltert, der Rückstand wird mit Diethylether ausgeschüttelt und nach Zugabe von Eis zu dem Filtrat wird die organische Phase mehrmals mit Salzlösung neutral gewaschen. Nach Abdampfen des Lösungsmittels bei niedriger Temperatur erhält man einen gelben Rückstand, der über Kieselgel mit Petrolether / Ethylacetat (8:2) gereinigt wird und nach Abziehen des Lösungsmittels einen hellgelben Sirup eines isomeren Gemisches der intermediär gebildeten Azidonitrate der Verbindungen der Formel III ergibt. Dieses Gemisch (17.8 g, 19.7 mmol) wird in 200 ml Dioxan gelöst und eine Lösung von Natriumnitrit (17.8 g) in 70 ml Wasser hinzugegeben. Die Mischung wird kräftig bei 85 °C gerührt. Nach 7 bis 8 Stunden kommt die Reaktion zum Stillstand (Überprüfung mit TLC). Die Mischung wird auf Eis gegossen und mit Diethylether extrahiert. Die organische Phase wird mit Wasser neutral gewaschen, über Magnesiumsulfat getrocknet, eingedampft und mittels Flashchromatographie (Petrolether / Ethylacetat 3 : 1) aufgereinigt. Man erhält als gelben Sirup 8.6 g (51 %)

**2-Azido-3,6-di-O-benzyl-4-O(3-O-allyl-2,4,6-tri-O-benzyl-β-D-galactopyranosyl)-2-deoxy-β-D-glucopyranose,**

eine Verbindung der Formel III.

$R_F$-Wert (TLC): 0.18 (Petrolether/Ethylacetat 3 : 1);

$[\alpha]_D$ = +10.8 (c = 1, Chloroform).

**Beispiel 6:**

Zu einer Lösung von der in Beispiel 5 hergestellten Verbindung (6.7 g, 7.8 mmol) in trockenem Dimethylformamid fügt man Imidazol (800 mg, 11.7 mmol) und tert-Butyldimethylsilylchlorid (1.53 g, 10.2 mmol). Die Lösung wird bei Raumtemperatur etwa 18 Stunden lang gerührt. Zur Umsetzung von noch vorhandenem Ausgangsmaterial werden dem Reaktionsansatz tert-Butyldimethyl-silyl-trifluormethansulfonat (2.06 g, 7.8 mmol) und erneut Imidazol (800 mg, 11.7 mmol) hinzugegeben und die Mischung wird für weitere 18 Stunden bei 60 °C gerührt. Danach wird die Mischung eingeengt, mit Dichlormethan verdünnt, mehrmals mit kaltem Wasser gewaschen, über Natriumsulfat getrocknet und erneut eingeengt. Das Rohprodukt wird über Kieselgel chromatographisch gereinigt (Petrolether / Ethylacetat 3 : 1) und das Epimerengemisch (gluco/manno) wird mittels Flashchromatographie (Petrolether / Ethylacetat 9 : 1) getrennt. Man erhält als farblose viskose Flüssigkeit (3.5 g, 46 %)
**tert-Butyldimethylsilyl-4-O-(3-O-allyl-2,4,6-tri-O-benzyl-β-D-galactopyranosyl)-2-azido-3,6-di-O-benzyl-2-deoxy-β-D-glucopyranosid,**
eine Verbindung der Formel IV.

$R_F$-Wert (TLC): 0.20 (Petrolether/Ethylacetat 9 : 1);
$[\alpha]_D$ = -17.2 (c = 1, Chloroform).

**Beispiel 7:**

Zu einer Lösung von der in Beispiel 6 hergestellten Verbindung (4.9 g, 5.0 mmol) in Ethanol / Toluol / Wasser (360 ml, 8 : 3 : 1) fügt man tris(Triphenylphosphin)-rhodium-I-chlorid (0.69 g, 0.75 mmol) und erhitzt die Mischung unter Rückfluß 2 Stunden lang. Die leicht gelbe Lösung wird eingeengt, mit 300 ml Dichlormethan verdünnt, die organische Lösung neutral gewaschen und eingedampft. Der Rückstand wird in Aceton/Wasser (200 ml, 10 : 1) aufgenommen und es werden HgO (162 mg, 0.75 mmol) und $HgCl_2$ (6.8 g, 25.0 mmol) hinzugefügt. Die Mischung wird 1 Stunde lang bei Raumtemperatur gerührt. Danach wird das Lösungsmittel abgezogen und der Rückstand in 400 ml Dichlormethan aufgenommen. Die organische Phase wird mehrmals mit Wasser, wäßriger Kaliumjodid-Lösung und wieder mit Wasser bis zur Neutralität gewaschen, über Magnesiumsulfat getrocknet und eingedampft. Der braune Rückstand wird mittels Flashchromatographie (Petrolether / Ethylacetat 8 : 2) aufgereinigt. Man erhält als Sirup (4.0 g, 86 %)
**tert-Butyldimethylsilyl-2-azido-3,6-di-O-benzyl-(2,4,6-tri-O-benzyl-β-D-galactopyranosyl)-2-deoxy-β-D-glucopyranosid,**
eine Verbindung der Formel IV.

$R_F$-Wert (TLC): 0.50 (Petrolether/Ethylacetat 3 : 1);
$[\alpha]_D$ = -18.6 (c = 1, Chloroform).

**Beispiel 8:**

Die nach Beispiel 7 hergestellte, sorgfältig getrocknete Verbindung (2.06 g, 2.21 mmol) und α-Galactopyranosyl-trichloracetimidat (3.025 g, 4.415 mmol) in trocknem Diethylether (44 ml) werden bei -20 °C unter einer Argon-Schutzgasatmosphäre tropfenweise mit einer Lösung von Trimethylsilyl-trifluormethansulfonat (0.1 M, 0.5 ml mit 1 ml Diethylether verdünnt) versetzt. Nach 5 Stunden wird festes Natriumhydrogencarbonat hinzugegeben und die Mischung wird filtriert und eingedampft. Der Rückstand wird mittels Flashchromatographie (Petrolether/Ethylacetat 8 : 2) aufgereinigt. Man erhält als Sirup (2.4 g, 75 %)
**tert-Butyldimethylsilyl-O-(2,3,4,6-tetra-O-benzyl-α-D-galactopyranosyl)-(1→3)-O-(2,4,6-tri-O-benzyl-β-D-galactopyranosyl)-(1→4)-O-2-azido-3,6-di-O-benzyl-2-deoxy-β-D-glucopyranosid,**
eine Verbindung der Formel VI.

$R_F$-Wert (TLC): 0.23 (Petrolether/Ethylacetat 6 : 4);
$[\alpha]_D$ = +17.5 (c = 1, Chloroform).

**Beispiel 9:**

Zu der nach Beispiel 8 hergestellten, sorgfältig getrockneten Verbindung (1.82 g, 1.25 mmol) in trockenem Tetrahydrofuran (40 ml) gibt man tropfenweise bei - 20 °C unter Argon eine Lösung von Tetrabutylammoniumfluorid (0.1 M, 2.5 ml verdünnt mit 5 ml Tetrahydrofuran). Nach 2 Stunden wird die Mischung auf Eis gegeben und intensiv mit Diethylether extrahiert. Die organische Phase wird mit Salzlösung und mit Wasser neutral gewaschen, über Magnesiumsulfat getrocknet, eingeengt und chromatographiert (Petrolether/ Ethylacetat 7 : 3). Der erhaltene getrocknete gelbe Sirup (1.4 g, 1.05 mmol) wird in trockenem Dichlormethan (35 ml) aufgenommen, und unter Argon werden Trichloracetonitril (1.5 ml, 15.8

mmol) und Natriumhydrid (10 x 25 mg, 1.05 mmol) hinzugefügt. Die Suspension wird 5 Stunden lang bei Raumtemperatur gerührt, filtriert, eingeengt und mittels Flashchromatographie (Petrolether/Ethylacetat 8 : 2) aufgereinigt. Man erhält als gelben Sirup (1.1 g, 70 %)
**O-(2,4,4,6-tetra-O-benzyl-α-D-galactopyranosyl)-(1→3)-O-(2,4,6-tri-O-benzyl-β-D-galactopyranosyl)-(1→4)-O-2-azido-3,6-di-O-benzyl-2-deoxy-β-D-glucopyranosyl-trichloracetimidat,**
eine Verbindung der Formel VI.

$R_F$-Wert (TLC): 0.48 (Petrolether/Ethylacetat 7 : 3);
$[\alpha]_D$ = +30.0 (c = 1, Chloroform).

**Beispiel 10:**

Zu einer Lösung von der nach Beispiel 9 hergestellten, gut getrockneten Verbindung (2.93 g, 1.97 mmol) und 8-Ethoxy-carbonyloctanol (800 mg, 4.0 mmol) in Dichlormethan / n-Hexan (36 ml, 1 : 1) gibt man unter Argon bei -15 °C eine Lösung von Bortrifluorid-Diethylether (0.1 M, 16.5 ml in 3 ml Dichlormethan/n-Hexan 1 : 2). Nach etwa 5 Stunden wird die Lösung durch Zugabe von Natriumhydrogencarbonat neutralisiert, filtriert und im Vakuum eingedampft, und der Rückstand wird wie beschrieben, chromatographiert (Petrolether/Diethylether 6 : 4). Man erhält als Sirup (2.6 g, 85 %)
**8-Ethoxycarbonyloct-1-yl-O-(2,3,4,6-tetra-O-benzyl-α-D-galactopyranosyl)-(1→3)-O-(2,4,6-tri-O-benzyl-β-D-galactopyranosyl)-(1→4)-O-2-azido-3,6-di-O-benzyl-2-deoxy-β-D-glucopyranosid,**
eine Verbindung der Formel I.

**Beispiel 11:**

Zu einer Mischung aus der Verbindung, hergestellt nach Beispiel 10 (246 mg, 0.16 mmol) in Dioxan (2 ml) und 50 ml einer Lösung von 4 % $NiCl_2$ x $6H_2O$ und 2 % $H_3BO_3$ in Ethanol wird tropfenweise eine gesättigte Lösung von Natriumborhydrid in Ethanol gegeben bis kein Ausgangsmaterial mehr vorhanden ist (ca. 8 h). Dan gibt man Essigsäureanhydrid (1.5 ml) hinzu und rührt noch 2 Tage bei 4 °C. Die Suspension wird mit Eiswasser verdünnt und mehrmals mit Dichlormethan extrahiert. Die vereinigten Extrakte werden mit gesättigter Natriumhydrogencarbonat-Lösung und Wasser neutral gewaschen, über Magnesiumsulfat getrocknet und im Vakuum eingedampft. Nach Aufreinigung über MPLC (Eluent: Petrolether / Ethylacetat 6 : 4) erhält man 242 mg (100 %) eines Sirups von
**8-Ethoxycarbonyloct-1-yl-O-(2,3,4,6-tetra-O-benzyl-α-D-galactopyranosyl)-(1→3)-O-(2,4,6-tri-O-benzyl-β-D-galactopyranosyl)-(1→4)-O-2-acetamido-3,6-di-O-benzyl-2-deoxy-β-D-glucopyranosid,**
eine Verbindung der Formel I.

$R_F$-Wert (TLC): 0.28 (Petrolether/Ethylacetat 6 : 4).

**Beispiel 12**

Zu einer Lösung von der nach Beispiel 11 hergestellten Verbindung (83 mg, 54 umol) in Etylacetat / Ethanol / Wasser / Essigsäure (11 ml, 7 : 3 : 1 : 0.05) werden katalytische Mengen von Pd / C hinzugegeben und die Mischung wird mit Wasserstoff reduziert. Nach etwa 12 Stunden ist die Reaktion beendet und die Mischung wird filtriert, im Vakuum eingeengt und mittels MPLC (Dichlormethan / Methanol 1 : 1) aufgereinigt. Man erhält nach Abziehen des Lösungsmittels als amorphes Material (30 mg, 77 %)
**8-Ethoxycarbonyloct-1-yl-O-α-galactopyranosyl-(1→3)-β-D-galactopyranosyl-(1-4)-O-2-acetamido-2-deoxy-β-D-glucopyranosid,**
eine Verbindung der Formel I;
$R_F$-Wert (TLC): 0.16 (Ethylacetat / Methanol / Wasser 8 : 2 : 1).

**Beispiel 13:**

Zu einer Lösung von der in Beispiel 5 hergestellten Verbindung (500 mg, 0.58 mmol) in trockenem Dichlormethan (20 ml) fügt man unter Argon Trichloracetonitril (0.58 ml, 5.8 mmol) und Natriumhydrid (6x 13.9 mg, 0.58 mmol) hinzu. Nach ca. sechsstündigem Rühren bei Raumtemperatur, wird die Suspension gefiltert, eingeengt und über $SiO_2$ gefiltert (Petrolether/Ethylacetat 6 : 4). Das entstandene Epimerengemisch (gluco/manno) wird mittels MPLC (Petrolether / Diethylether 3 : 2) aufgereinigt. Man erhält 280 mg (48 %) von
**O-[4-O-(3-O-Allyl-2,4,6-tri-O-benzyl-β-D-galactopyranosyl)-2-azido-3,6-di-O-benzyl-2-deoxy-α-D-glucopyranosyl]-trichloracetimidat,**

eine Verbindung der Formel V;
Mp.: 66 - 67 °C; $R_F$-Wert (TLC): 0.51 (Petrolether / Ethylacetat 3 : 2);
$[\alpha]_D$ = + 27.1 (c = 1, Chloroform).

**Beispiel 14:**

Zu einer Lösung von der in Beispiel 13 hergestellten Verbindung (200 mg, 0.20 mmol) und 8-Ethoxycarbonyloctanol (98 mg, 0.20 mmol) in Dichlormethan / n-Hexan (10 ml, 1: 4) gibt man unter Argon bei - 20 °C eine Lösung von Bortrifluorid Diethylether (0.1 M) in Dichlormethan (0.5 ml in 2 ml n-Hexan). Nach 3 Stunden wird die Lösung durch Zusatz von festem Natriumhydrogencarbonat neutralisiert, filtriert und im Vakuum eingedampft. Der Rückstand wird durch Filration über $SiO_2$ und mittels MPLC (Petrolether / Ethylacetat 4 : 1) gereinigt. Man erhält in reiner Form (115 mg, 55 %)
**8-Ethoxycarbonyloct-1-yl-O-2-azido-3,6-di-O-benzyl-4-O-(3-O-allyl-2,4,6,-tri-O-benzyl-β-D-galactopyranosyl)-2-deoxy-β-D-glucopyranosid,**
eine Verbindung der Formel VII;
$R_F$-Wert (TLC): 0.32 (Petrolether/Ethylacetat 4 : 1);
$[\alpha]_D$ = -14.8 (c = 1, Chloroform).

**Beispiel 15:**

Die nach Beispiel 14 hergestellte Verbindung (535 mg, 0.513 mmol) wird in Toluol / Ethanol / Wasser (30 ml, 3 : 8 : 2) gelöst und der Lösung tris-(Triphenylphosphin)-rhodium-I-chlorid (72 mg, 0.08 mmol) hinzugefügt. Nach etwa drei Stunden unter Rückfluß ist kein Ausgangsmaterial mehr vorhanden. Die Mischung wird eingedampft, in Dichlormethan aufgenommen, mit Wasser gewaschen, wieder eingedampft und in Aceton / Wasser (20 ml, 10 : 1) gelöst. Dann werden HgO (17 mg, 0.078 mmol) und $HgCl_2$ (706 mg, 2.60 mmol) hinzugefügt. Nach einer Stunde bei Raumtemperatur wird die Mischung eingeengt, mit Dichlormethan verdünnt, die organische Phase mit Wasser und Kaliumjodidlösung gewaschen, getrocknet und eingedampft. Der Rückstand wird mittels Flashchromatographie (Petrolether / Ethylacetat 8 : 2) aufgereinigt. Man erhält als viskose Flüssigkeit (455 mg, 89 %)
**8-Ethoxycarbonyloct-1-yl-O-2-azido-3,6-di-O-benzyl-4-O-(2,4,6,-tri-O-benzyl-β-D-galactopyranosyl)-2-deoxy-β-D-glucopyranosid,**
eine Verbindung der Formel VII;
$R_F$-Wert (TLC): 0.43 (Petrolether/Ethylacetat 7 : 3);
$[\alpha]_D$ = -17.7 (c = 1, Chloroform).

**Beispiel 16:**

Zu einer Lösung von der nach Beispiel 15 hergestellten Verbindung (141 mg, 0.141 mmol) und α-Galactopyranosyl-trichloracetimidat (116 mg, 0.17 mmol) in trockenem Diethylether (3 ml) gibt man tropfenweise bei Raumtemperatur und einer Argon-Atmosphere eine Lösung von Trimethylsilyl-trifluormethansulfonat (0.1 M, 0.2 ml verdünnt mit 1 ml Diethylether). Nach 3 Stunden fügt man festes Natriumhydrogencarbonat hinzu, filtriert ab und engt ein. Die Reinigung des Rückstandes erfolgt mittels MPLC (Petrolether / Diethylether 1 : 1). Man erhält eine Gemisch aus (170 mg, 79 %)
**8-Ethoxycarbonyloct-1-yl-O-(2,3,4,6-tetra-O-benzyl-α-D-galactopranosyl)-(1→3)-O-(2,4,6-tri-O-benzyl-β-D-galactopyranosyl)-(1→4)-O-(2-azido-3,6-di-O-benzyl-2-deoxy-β-D-glucopyranosid),**
eine Verbindung der Formel I und
**8-Ethoxycarbonyloct-1-yl-O-(2,3,4,6-tetra-O-benzyl-β-D-galactopyranosyl)-(1→3)-O-(2,4,6-tri-O-benzyl-β-D-galactopyranosyl)-(1→4)-O-(2-azido-3,6-di-O-benzyl-2-deoxy-β-D-glucopyranosid).**
Durch erneute MPLC können die Isomeren getrennt werden. Man erhält das entsprechende α-Isomer zu 61 % und das entsprechende unerwünschte β-Isomer zu 17 %.
$R_F$-Wert (TLC) α-Isomer: 0.21 (Petrolether/Ethylacetat 6 : 4)
$R_F$-Wert (TLC) β-Isomer: 0.28 (Petrolether/Ethylacetat 6 : 4).

**Beispiel 17:**

Eine Lösung, bestehend aus einer nach Beispiel 13 hergestellten Donorverbindung (200 mg, 0.20 mmol) und einer nach Beispiel 7 hergestellten Akzeptorverbindung (186 mg, 0.20 mmol) in etwas Dichlormethan (ca. 3 ml) wird unter Argon auf -20 °C abgekühlt und mit n-Hexan versetzt (8 ml). Die

Reaktion wird gestartet durch tropfenweise Zugabe einer Lösung von Trimethylsilyl-trifluormethansulfonat (0.1 M, Dichlormethan) in n-Hexan. Nach etwa einer Stunde wird die Lösung wie in den anderen Beispielen beschrieben gewaschen, neutralisiert, gefiltert und eingeengt. Der Rückstand wird über Kieselgel gefiltert und mittels MPLC (Petrolether / Ethylacetat 8 : 2) weiter aufgereinigt. Nach Abdampfung des Lösungsmittels erhält man als Sirup (255 mg, 72 %)
**tert-Butyldimethylsilyl-O-(3-O-allyl-2,4,6-tri-O-benzyl-β-D-galactopyranosyl)-(1→4)-O-(2-azido-3,6-di-O-benzyl-2-deoxy-β-D-glucopyranosyl)-(1→3)-O-(2,4,6-tri-O-benzyl-β-D-galactopyranosyl)-(1→4)-O-(2-azido-3,6-di-O-benzyl-2-deoxy-β-D-glucopyranosid),**
eine Verbindung der Formel VIII;
$R_F$-Wert (TLC): 0.37 (Petrolether/Ethylacetat 8 : 2);
$[\alpha]_D$ = -33.5 (c = 1, Chloroform).

## Patentansprüche

1. Verfahren zur stereoselektiven Herstellung von α(1-3), β(1-4) konfigurierten Trisacchariden der Formel I,

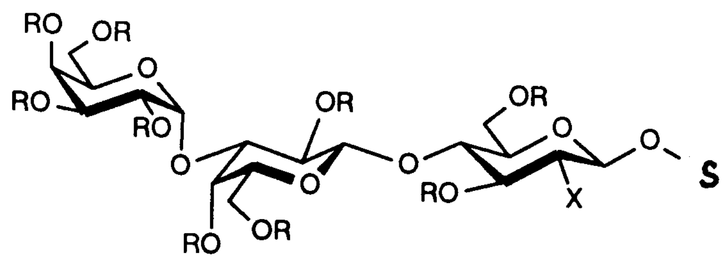

I

worin

R H, R'$CH_2$-,
R' H oder Alkyl mit 1 bis 3 C-Atomen oder unsubstituiertes oder mit Halogen, OH, Alkyl oder O-Alkyl substituiertes Phenyl,
S einen Spacer und
X $N_3$ oder -$NHCOCH_3$
bedeuten,

aus Lactose, dadurch gekennzeichnet, daß man
i. Lactose zu D-Lactal umsetzt;
ii. D-Lactal zu den neuen Lactalderivaten der Formel II,

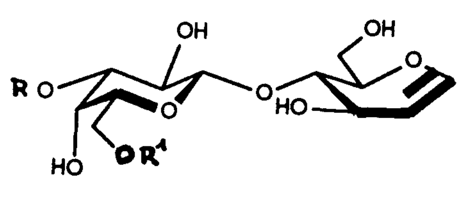

II

worin
R R'$CH_2$- oder -$CH_2$-CH=$CH_2$,
$R^1$ H, R'$CH_2$-, $CH_2$-CH=$CH_2$ oder R'-C(O)- und
R' H, Alkyl mit 1 bis 3 C-Atomen, oder
unsubstituiertes oder mit Halogen, OH, Alkyl oder O-Alkyl substituiertes Phenyl

bedeuten,

partiell alkyliert;
iii. die Verbindung der Formel II, worin R -$CH_2$-CH=$CH_2$ und $R^1$ H bedeuten, zu mit R'$CH_2$-vollständig geschützten Mono-Allyl-Verbindungen umsetzt;

iv. diese stereoselektiv durch Azidonitratisierung zu den Azido-Lactosederivaten der Formel III,

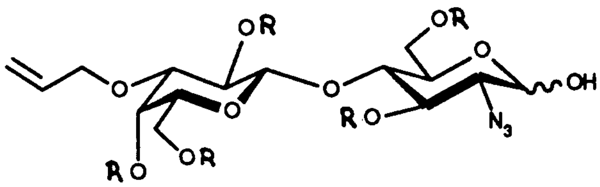

III

worin R $R'CH_2$- bedeutet und R' die angegebene Bedeutung hat, umsetzt;
v. die Verbindungen der Formel III entweder zu den Azido-Lactosederivaten der Formel IV,

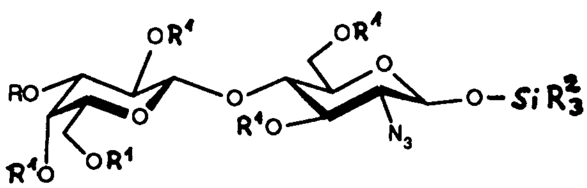

IV

worin die zunächst entstehende Allylverbindung durch Abspaltung der Allylgruppe zur OH-freien Verbindung umgesetzt wird und der Silylrest β-konfiguriert ist, oder zu aktivierten Azido-Lactosederivaten der Formel V,

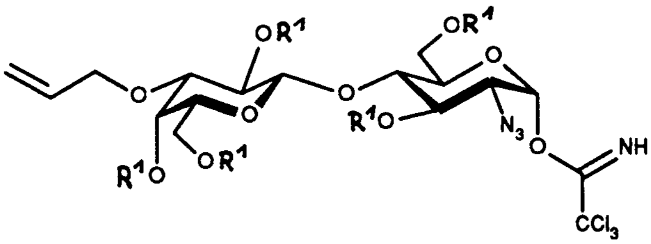

V

worin der Trichloracetimidat-Rest ($O-C(=NH)CCl_3$) vorzugsweise α-konfiguriert ist, stereoselektiv umsetzt, wobei in den Formeln IV und V $R^1$ $R'CH_2$-, R Allyl oder H und $R^2$ $C_1$ bis $C_4$-Alkyl oder Phenyl bedeuten und R' die angegebenen Bedeutung hat;
vi. die Disaccharide der Formel IV oder V unter Einführung eines Galactopyranosyl-Restes und Substitution des glykosidischen Restes des Glucopyranosylringes durch einen Spacerrest -O-S zu den Verbindungen der Formel I, worin

R $R'CH_2$-, X $N_3$ und S einen Spacer

bedeuten,

R' die angegebene Bedeutung hat und der Spacerrest β-konfiguriert ist,

umsetzt, und
vii. gegebenenfalls die Azido-Gruppe zur $-NHCOCH_3$ und die OR-Reste zu OH-Gruppen reduziert.

**2.** Verfahren nach Anspruch 1, dadurch gekennzeichnet, daß man den Verfahrensschritt ii aus Anspruch 1 in Gegenwart von Dibutyl-zinnoxid ausführt.

**3.** Verfahren nach Anspruch 1 oder 2, dadurch gekennzeichnet, daß man gemäß Verfahrensschritt vi aus Anspruch 1 die Disaccharide der Formel IV mit α-Galactopyranosyltrichloracetimidat stereoselektiv zu den α(1-3), β(1-4) konfigurierten Verbindungen der Formel VI

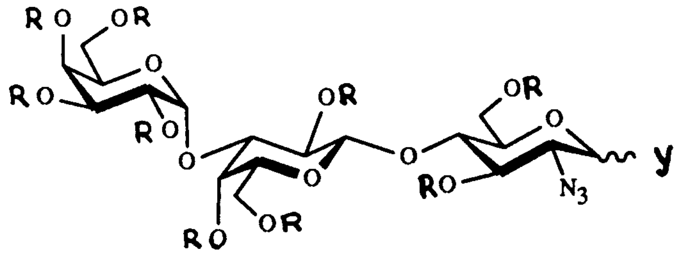

VI

umsetzt und anschließend die zunächst vorliegende $\beta$-ständige O-Silyl- durch die $\alpha$-ständige OC-(=NH)$CCl_3$-Gruppe austauscht und die so erhaltenen Verbindungen mit einem Spacer-Reagens zu den Verbindungen der Formel I umsetzt, wobei in der Formel VI R R'$CH_2$-, Y $\beta$-$OSiR^2_3$ oder $\alpha$-OC(=NH)-$CCl_3$ und R' und $R^2$ die angegebenen Bedeutungen haben.

**4.** Verfahren nach Anspruch 1 oder 2, dadurch gekennzeichnet, daß man gemäß Verfahrensmerkmal vi aus Anspruch 1 die Disaccharide der Formel V mit einem Spacer-Reagens zu Verbindungen der Formel VII,

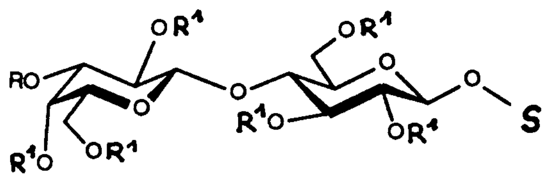

VII

worin $R^1$ R'$CH_2$-, R Allyl oder H, X $N_3$ oder -$NHCOCH_3$ und S einen Spacer bedeuten und R' die angegebene Bedeutung hat, umsetzt, wobei die zunächst entstehende Allylverbindung durch Abspaltung der Allylschutzgruppe zur OH-freien Verbindung entschützt wird und diese Verbindung mit $\alpha$-Galactopyranosyl-trichloracetimidat zu den Verbindungen der Formel I umsetzt.

**5.** Verfahren nach einem der Ansprüche 1 bis 4, dadurch gekennzeichnet, daß man als Spacer-Reagens eine Verbindung der Formel S-OH worin S -$(CH_2)_n$-COOR" $C_1$ bis $C_4$-Alkyl und n eine ganze Zahl von 4 bis 12 bedeuten, einsetzt.

**6.** Neue Lactalderivate der Formel II nach Anspruch 1.

**7.** Verfahren zur Herstellung der Verbindungen der Formel II nach Anspruch 1 bzw. 6, dadurch gekennzeichnet, daß man Glykale, vorzugsweise D-Lactal, mit Dibutyl-Zinnoxid und R-Hal umsetzt, wobei R Allyl oder R'$CH_2$- und Hal Cl, Br, F, J bedeuten und R' die angegebene Bedeutung hat.

**8.** Neue $\alpha$(1-3), $\beta$(1-4) konfigurierte Trisaccharide der Formel VI nach Anspruch 3.

**9.** Neue $\beta$(1-4) konfigurierte Disaccharide der Formel VII nach Anspruch 4 worin S einen Spacer der Formel -$(CH_2)_n$-COOR" bedeutet und R" die angegebene Bedeutung hat.

**10.** Neue $\beta$-verknüpfte Oligolactosamine der Formel VIII,

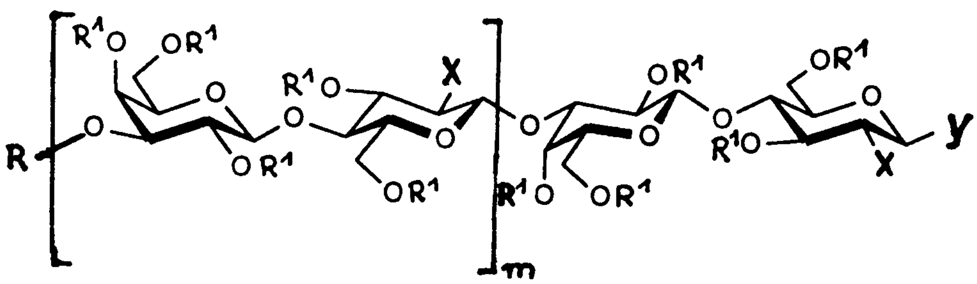

VIII

worin

| | |
|---|---|
| R | H, Allyl, |
| $R^1$ | H, $R'CH_2$-, |
| X | $N_3$, $-NHCOCH_3$, |
| Y | $\beta$-$OSiR^2_3$, $\alpha$-O-C(=NH)$CCl_3$, |
| $R^2$ | $C_1$- bis $C_4$-Alkyl oder Phenyl und |
| m | eine ganze Zahl von 1 bis 4 |

bedeuten und R' die angegebene Bedeutung hat.

**11.** Verfahren zur stereoselektiven Herstellung der Verbindungen der Formel VIII nach Anspruch 10 aus Lactose, dadurch gekennzeichnet, daß man die Verfahrensschritte i-v nach Anspruch 1 ausführt und eine Verbindung der Formel IV, worin R H bedeutet, mit einer Verbindung der Formel V mittels einer Lewis-Säure, vorzugsweise Trimethylsilyltrifluormethansulfonat zu einem Tetrasaccharid der Formel VIII (m = 1) umsetzt und dieses gegebenenfalls mit einer oder mehreren Verbindungen der Formel V in analoger Weise zu den entsprechenden Oligosacchariden (m > 1) reagieren läßt.

Europäisches Patentamt

# EUROPÄISCHER RECHERCHENBERICHT

Nummer der Anmeldung

EP 92109711.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| A | WO - A - 90/01 488 (SYMBICOM) * Ansprüche 1-8; Beispiele * -- | 1-11 | C 07 H 3/06<br>C 07 H 3/04 |
| D,A | CARBOHYDRATE RESEARCH, Band 135, Nr. 1, 15. Dezember 1984, Amsterdam, A. MARANDUBA et al. "Glycosylation of Lactose" Seiten 330-336 * Gesamt * -- | 1-11 | |
| D,A | CARBOHYDRATE RESEARCH, Band 136, 28. Februar 1985, Amsterdam, P.J. GAREGG et al. "A Synthesis of 8-Methoxycarbonyloct-1-yl O-alpha-D-Galactophyranosyl-(1-3)-O-beta-D-Galactopyranosyl-(1-4)-2-Acetamido-2-Deoxy-beta-D-Glucopyranoside" Seiten 207-213 * Gesamt * -- | 1 | **RECHERCHIERTE SACHGEBIETE (Int. Cl.⁵)**<br>C 07 H 3/00<br>C 07 H 15/00 |
| D,A | CARBOHYDRATE RESEARCH, Band 56, Nr. 1, Juni 1977, Amsterdam, M.A. NASHED et al. "O-Benzylated Thio Sugars" Seiten 325-336 * Gesamt * -- | 1,2,6 | |
| D,A | LIEBIGS ANNALEN DER CHEMIE, Heft 1, Januar 1989, Weinheim, K.H. JUNG et al. "Selectively Protected Lactose and 2-Azido Lactose, Building Blocks for | 1,4 | |

Der vorliegende Recherchenbericht wurde für alle Patentansprüche erstellt.

| Recherchenort | Abschlußdatum der Recherche | Prüfer |
|---|---|---|
| WIEN | 24-09-1992 | IRMLER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-

EP 92109711.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| | Glycolipid Synthesis"<br>Seiten 1099-1106<br>* Seiten 1099-1101 *<br>---- | | |

RECHERCHIERTE SACHGEBIETE (Int. Cl.5)

Der vorliegende Recherchenbericht wurde für alle Patentansprüche erstellt.

| Recherchenort | Abschlußdatum der Recherche | Prüfer |
|---|---|---|
| WIEN | 24-09-1992 | IRMLER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82